# FASCICULE DE BREVET EUROPEEN

(11) **EP 4 637 536 B1**
(45) Date de publication et mention de la délivrance du brevet: **28.10.2026**
(21) Numéro de dépôt: 23833747.1
(22) Date de dépôt: 18.12.2023
(51) Int. Cl.: A61B 5/024, A61B 5/00

(54) **PROCÉDÉ ET DISPOSITIF AMÉLIORÉ D'ÉVALUATION DES BESOINS EN COMPOSÉ MÉDICAMENTEUX D'UN PATIENT, ET DISPOSITIF DE DÉLIVRANCE ASSOCIÉ**
VERBESSERTE VORRICHTUNG UND VERFAHREN ZUR BEURTEILUNG DES BEDARFS EINER ARZNEIMITTELVERBINDUNG EINES PATIENTEN UND ZUGEHÖRIGE ABGABEVORRICHTUNG
IMPROVED DEVICE AND METHOD FOR ASSESSING DRUG COMPOUND NEEDS OF A PATIENT AND ASSOCIATED DELIVERY DEVICE

(30) Priorité: 21.12.2022 FR 2214151
(43) Date de publication de la demande: 29.10.2025
(73) Titulaire: Université de Lille, 59800 Lille (FR); Centre Hospitalier Universitaire de Lille, 59000 Lille (FR); Institut National de la Santé et de la Recherche Médicale (INSERM), 75013 Paris (FR)
(72) Inventeur: DE JONCKHEERE, Julien, 59160 Lome (FR); LOGIER, Régis, 59520 Marquette Lez Lille (FR); IBARISSENE, Idir, 59000 Lille (FR); DELECROIX, Michel, 59139 Wattignies (FR); JEANNE, Mathieu, 59800 Lille (FR)
(74) Mandataire: Santarelli
(86) Numéro de dépôt international: PCT/EP2023/086345
(87) Numéro de publication internationale: WO 2024/133078

(56) Documents cités:
- FR-A1- 3 006 880
- GONZALEZ-CAVA JOSE M. ET AL: "A Novel Fuzzy Algorithm to Introduce New Variables in the Drug Supply Decision-Making Process in Medicine", COMPLEXITY., vol. 2018, 1 January 2018 (2018-01-01), US, pages 1 - 15, XP093054087, ISSN: 1076-2787, DOI: 10.1155/2018/9012720
- SCHUBERT A U ET AL: "A fuzzy system for regulation of the analgesic remifentanil during general anaesthesia", CONTROL AND AUTOMATION, 2008 16TH MEDITERRANEAN CONFERENCE ON, IEEE, PISCATAWAY, NJ, USA, 25 June 2008 (2008-06-25), pages 1634 - 1639, XP031308501, ISBN: 978-1-4244-2504-4
- SIMANSKI O ET AL: "Progress of automatic drug delivery in anaesthesia-The 'Rostock assistant system for anaesthesia control (RAN)'", INTERNATIONAL JOURNAL OF ADAPTIVE CONTROL AND SIGNALPROCESSING, WILEY, CHICHESTER, GB, vol. 23, no. 5, 17 September 2008 (2008-09-17), pages 504 - 521, XP071734148, ISSN: 0890-6327, DOI: 10.1002/ACS.1068

## Description

### DOMAINE TECHNIQUE DE L'INVENTION

Le domaine de l'invention est celui du domaine de la prise en charge de la douleur d'un patient.

Plus précisément, l'invention concerne un procédé amélioré d'évaluation des besoins en composé médicamenteux d'un patient. L'invention concerne également un dispositif amélioré d'évaluation des besoins en composé médicamenteux d'un patient, ainsi qu'un dispositif de délivrance de composés médicamenteux comprenant notamment un tel dispositif d'évaluation. Le composé médicamenteux est en particulier un composé analgésique et/ou hypnotique.

L'invention trouve notamment des applications dans la prise en charge de la douleur d'un patient lors d'une intervention chirurgicale d'un patient sous anesthésie générale ou locale. Selon d'autres possibilités, l'invention pourra trouver des applications plus largement pour la prise en charge de la douleur de patients conscients ou inconscients dans les hôpitaux, notamment en réanimation ou en soin palliatifs, dans les structures de soins, ou à domicile.

### ÉTAT DE LA TECHNIQUE

Dans le cadre de la prise en charge de la douleur d'un patient, il est nécessaire d'évaluer précisément et en temps réel le niveau de la douleur, de manière à pouvoir apporter une réponse médicale adaptée aux besoins du patient.

Par exemple, la réponse médicale peut consister à un injecter un composé analgésique, antalgique ou anti-douleur au patient.

La quantité de composé injecté au patient doit toutefois être proportionnée, et être personnalisée pour le patient selon son état et ses caractéristiques physiologiques.

Afin d'évaluer le niveau de douleur d'un patient, il est connu la détermination d'un indice dit ANI (acronyme de « Analgesia Nociception Index »), déterminé en fonction de la variabilité de la fréquence cardiaque du patient. La détermination d'un tel indice ANI est connu de la technique, notamment au moyen des dispositifs et procédés connus des documents de brevet FR 2821460, FR 2840187, FR 2864388 et EP 1804655 déposés par le Demandeur.

Afin d'évaluer le besoin en composés analgésiques et/ou hypnotiques en temps réel et en tenant compte de l'évolution dans le temps du niveau de douleur du patient, ainsi que du type de douleur ressenti par le patient, le Demandeur a mis au point un dispositif et procédé d'évaluation des besoins en composés analgésiques et/ou hypnotiques qui tient compte de moyennes de l'indice ANI sur des périodes courte et longue, ainsi que de la pente de l'indice ANI sur ces périodes.

Ces techniques ont notamment fait l'objet des brevets FR 3006879 et FR 3006880 de la part du Demandeur.

Les techniques précitées prévoient la détermination d'une quantité de composé analgésique et/ou hypnotique à administrer ponctuellement au patient. Une telle quantité de composé, importante en comparaison à la quantité de composé normalement administrée, et destinée à être administrée sur une période courte, est appelée « bolus » en terminologie médicale.

Il est toutefois apparu le besoin d'une technique d'évaluation des besoins en composé médicamenteux qui soit plus stable et répétible, notamment en ce qui concerne la détermination d'un bolus à administrer au patient.

Il est également apparu le besoin de déterminer plus finement, c'est-à-dire de manière plus précise et plus proche des besoins instantanés en composé du patient, la quantité de composé que nécessite un patient. En effet, certaines techniques connues sous-évaluent ou sur-évaluent les besoins en composé, ce qui se traduit en un risque accru d'hypotension ou hypertension du patient.

Les documents [GONZALEZ-CAVA JOSE M. ET AL: "A Novel Fuzzy Algorithm to Introduce New Variables in the Drug Supply Decision-Making Process in Medicine", 2018], [SCHUBERT A U ET AL: "A fuzzy system for regulation of the analgesic remifentanil during general anaesthesia", 2008] et [SIMANSKI O ET AL: "Progress of automatic drug delivery in anaesthesia-The 'Rostock assistant system for anaesthesia control (RAN)"', 2008] décrivent des procédés basés sur la logique floue pour contrôler l'administration d'analgésiques.

### EXPOSÉ DE L'INVENTION

La présente invention vise à remédier à tout ou partie des inconvénients de l'état de la technique cités ci-dessus.

À cet effet, l'invention vise un procédé d'évaluation des besoins en composé médicamenteux d'un patient, destiné à être mis en œuvre par un dispositif d'évaluation comprenant des moyens de mesure de la variabilité cardiaque du patient entre 0.15Hz et 4Hz, une unité de traitement et d'enregistrement de données, et des moyens d'enregistrement de données physiologiques relatives au patient, le procédé comprenant les étapes suivantes :
- une étape de mesure de la variabilité cardiaque du patient entre 0.15Hz et 4Hz;
- une étape de première détermination par l'unité de traitement et d'enregistrement de données, à partir des données de variabilité cardiaque mesurées, d'un indice ANI relatif au niveau de douleur du patient ;
- une étape de deuxième détermination par l'unité de traitement et d'enregistrement de données, à partir de l'indice ANI, d'un indice MC, représentatif de la moyenne de l'indice ANI sur une période dite courte, et d'un indice ML, représentatif de la moyenne de l'indice ANI sur une période dite longue, ainsi que les pentes PC et PL respectivement desdits indices MC et ML, les indices MC et ML correspondant au niveau de douleur d'un patient et les indices PC et PL à son évolution, respectivement sur une période courte et une période longue;
- une étape d'enregistrement de données physiologiques relatives au patient, par les moyens d'enregistrement de données physiologiques relatives au patient ;
- une étape de troisième détermination par l'unité de traitement et d'enregistrement de données, en fonction des indices MC et ML, de leurs pentes PC et PL, et des données physiologiques relatives au patient, le besoin en composé médicamenteux nécessité par le patient, ladite étape de troisième détermination comprenant une sous-étape de détermination d'une quantité dudit composé à administrer ponctuellement au patient, dit bolus, comprenant :
- la détermination d'une première variable de logique floue *C_{MC}*, comprise entre 0 et 1, à partir d'une fonction d'appartenance F_{MC} de l'indice MC,
- la détermination d'une deuxième variable de logique floue *C_{PC}*, comprise entre 0 et 1, à partir d'une fonction d'appartenance F_{PC} de l'indice PC,
- la détermination que le bolus est égal à une première quantité prédéterminée dudit composé lorsque la somme de la première variable de logique floue *C_{MC}* et de la deuxième variable de logique floue *C_{PC}* est strictement supérieure à 1, et que le bolus est égal à zéro sinon ;
ladite étape de troisième détermination comprenant en outre une sous-étape de détermination d'une deuxième quantité dudit composé à administrer, inférieure à ladite première quantité prédéterminée, déterminée en fonction des indices MC et ML, de leurs pentes PC et PL, et des données physiologiques relatives au patient, cette sous-étape étant exécutée par le procédé à la suite de la sous-étape de détermination d'un bolus lorsque le bolus est égal à zéro.

Le procédé selon l'invention repose sur une étape de logique floue, également appelée « fuzzy logic » en terminologie anglaise.

La détermination d'un bolus au moyen d'une sous-étape de logique floue permet d'améliorer fortement la stabilité et la répétabilité de l'évaluation en besoin en composé, en comparaison avec les techniques connues de la technique.

La sous-étape de détermination d'une quantité du composé à administrer ponctuellement au patient, c'est-à-dire une sous-étape de détermination d'un bolus, permet d'évaluer si un bolus peut être administré au patient ou non.

Si un bolus peut, au regard des valeurs seuil MC et PC, être administré au patient, la valeur du bolus correspond à une quantité prédéterminée de composé. La quantité prédéterminée de composé peut être fixe, ou être définie en amont de la mise en œuvre du procédé, par exemple en fonction de données relatives au patient.

Si un bolus ne peut pas, au regard des valeurs seuil MC et PC, être administré au patient, la valeur du bolus est nulle.

Le calcul de la moyenne de l'indice ANI peut être effectué sur une fenêtre glissante. En particulier dans un tel cas, la répétabilité du procédé selon l'invention est encore plus importante en comparaison avec la technique connue.

Ces aspects permettent de fournir un procédé qui soit performant et sûr, et qui répond aux exigences pour la mise sur le marché d'un tel procédé mis en œuvre par un dispositif médical.

On précise ici que le procédé peut fonctionner en boucle, en temps réel ou périodiquement, c'est-à-dire que lesdites étapes du procédé sont répétées à la suite de façon à déterminer en temps réel ou périodiquement le besoin en composés médicamenteux du patient.

Dans le cadre d'essais cliniques du procédé selon l'invention, il a été observé une diminution de la quantité de composé antalgiques et/ou hypnotiques devant être administrés aux patients, et une meilleure stabilité hémodynamique, c'est-à-dire que les patients sont moins sujets à l'hypotension et à l'hypertension, en comparaison aux procédés connus.

Selon un deuxième aspect, l'invention a également pour objet un dispositif d'évaluation des besoins en composé médicamenteux d'un patient, comprenant des moyens de mesure de la variabilité cardiaque du patient entre 0.15Hz et 4Hz, une unité de traitement et d'enregistrement de données , et des moyens d'enregistrement de données physiologiques relatives au patient, l'unité de traitement et d'enregistrement comprenant un processeur et une mémoire informatique stockant des instructions configurant le processeur pour :
- recevoir des données de la variabilité cardiaque du patient entre 0.15Hz et 4Hz ;
- déterminer, à partir des données de variabilité cardiaque reçues, d'un indice ANI relatif au niveau de douleur du patient ;
- déterminer, à partir de l'indice ANI, d'un indice MC, représentatif de la moyenne de l'indice ANI sur une période dite courte, et d'un indice ML, représentatif de la moyenne de l'indice ANI sur une période dite longue, ainsi que les pentes PC et PL respectivement desdits indices MC et ML, les indices MC et ML correspondant au niveau de douleur d'un patient et les indices PC et PL à son évolution, respectivement sur une période courte et une période longue ;
- recevoir des données physiologiques relatives au patient ;
- déterminer, en fonction des indices MC et ML, de leurs pentes PC et PL, et des données physiologiques relatives au patient, le besoin en composé médicamenteux devant être administré audit patient, cette étape comprenant une sous-étape de détermination d'une première quantité dudit composé à administrer ponctuellement au patient, dit bolus, comprenant :
- la détermination d'une première variable de logique floue *C_{MC}*, comprise entre 0 et 1, à partir d'une fonction d'appartenance *F_{MC}* de l'indice MC,
- la détermination d'une deuxième variable de logique floue *C_{PC}*, comprise entre 0 et 1, à partir d'une fonction d'appartenance *F_{PC}* de l'indice PC,
- la détermination que le bolus est égal à une première quantité prédéterminée lorsque la somme de la première variable de logique floue *C_{MC}* et de la deuxième variable de logique floue *C_{PC}* est strictement supérieure à 1, et que le bolus est égal à zéro sinon
- déterminer une deuxième quantité dudit composé à administrer, inférieure à ladite première quantité prédéterminée, en fonction des indices MC et ML, de leurs pentes PC et PL, et des données physiologiques relatives au patient, à la suite de la détermination d'un bolus, lorsque le bolus est égal à zéro.

Un tel dispositif présente les avantages et effets techniques du procédé selon l'invention décrit ci-avant.

Le dispositif d'évaluation des besoins en composé médicamenteux d'un patient peut notamment se présenter sous la forme d'un ordinateur comportant des entrées auxquelles sont connectés un capteur de variabilité cardiaque et au moins un autre capteur de données physiologiques du patient.

Le dispositif d'évaluation peut comprendre un dispositif d'affichage de données, notamment d'une quantité de composé médicamenteux à administrer.

On précise que le procédé, et le dispositif, peuvent également être un procédé, et un dispositif, d'évaluation des besoins en composé médicamenteux, ou médicaments, et/ou en soins paramédicaux d'un patient. De façon préférée, le composé médicamenteux est un composé analgésique et/ou hypnotique.

D'autres caractéristiques particulièrement avantageuses et commodes du procédé selon l'invention, et du dispositif d'évaluation selon l'invention sont décrites ci-après.

Selon un mode de réalisation, dans le procédé et le dispositif d'évaluation selon l'invention, la fonction d'appartenance *F_{MC}* de l'indice MC est :
- égale à 1 lorsque l'indice MC est inférieur ou égal à une valeur seuil basse *S_{MCBas}*,
- égale à 0 lorsque l'indice MC est supérieur ou égal à une valeur seuil haute *S_{MCHaut}*,
- et présente une transition décroissante monotone entre 1 et 0, lorsque l'indice MC est compris entre les valeurs seuil basse *S_{MCBas}* et haute *S_{MCHaut}*, et dans lequel fonction d'appartenance *F_{PC}* de l'indice PC est :
- égale à 1 lorsque l'indice PC est inférieur ou égal à une valeur seuil basse *S_{PCBas}*,
- égale à 0 lorsque l'indice PC est supérieur ou égal à une valeur seuil haute *S_{PCHaut}*,
- et présente une transition décroissante monotone entre 1 et 0, lorsque l'indice PC est compris entre les valeurs seuil basse *S_{PCBas}* et haute *S_{PCHaut}*.

Le choix de ce type de fonction d'appartenance a permis de constater une bonne performance du procédé, qui est particulièrement stable et répétible.

Le type de transition décroissante monotone peut être adapté en fonction du type de composé destiné à être administré, et/ou en fonction de la situation clinique du patient. De même, les seuils hauts et bas peuvent être adaptés en fonction de ces mêmes critères.

Selon un mode de réalisation, dans le procédé et le dispositif d'évaluation selon l'invention, la fonction d'appartenance *F_{MC}* de l'indice MC est définie selon l'expression : ${F}_{MC} = \left\{\begin{matrix}1 , & si MC \leq {S}_{MCBas} \\ \frac{MC - {S}_{MCHaut}}{{S}_{MCBas} - {S}_{MCHaut}} , & si {S}_{MCBas} < MC < {S}_{MCHaut} \\ 0 , & si MC \geq {S}_{MCHaut}\end{matrix}\right.$ et la fonction d'appartenance *F_{PC}* de l'indice PC est définie selon l'expression : ${F}_{PC} = \left\{\begin{matrix}1 , & si PC \leq {S}_{PCBas} \\ \frac{PC - {S}_{PCHigh}}{{S}_{PCBas} - {S}_{PCHaut}} , & si {S}_{PCBas} < PC < {S}_{PCHaut .} \\ 0 , & si PC \geq {S}_{PCHaut}\end{matrix}\right.$

De manière générale, une transition linéaire décroissante permet une bonne performance du procédé.

Une telle transition est particulièrement adaptée lors d'une chirurgie pendant laquelle le patient est sous anesthésie générale.

Selon un mode de réalisation, dans le procédé et le dispositif d'évaluation selon l'invention :
- la valeur seuil *S_{MCBas}* est comprise entre 45 et 55, et préférentiellement égale à 50,
- la valeur seuil *S_{MCHaut}*est comprise entre 58 et 68, et préférentiellement égale à 63,
- la valeur seuil *S_{PCBas}* est comprise entre -40 et -30 et préférentiellement égale à -35,
- la valeur seuil *S_{PCHaut}* est comprise entre -30 et -20, et préférentiellement égale à -25.

Les fourchettes de seuils, ainsi que les seuils préférentiels, sont particulièrement adaptées lors d'une chirurgie pendant laquelle le patient est sous anesthésie générale.

Ces valeurs sont particulièrement adaptées lorsque le composé médicamenteux est un composé analgésique et/ou hypnotique qui est du rémifentanil.

Selon un mode de réalisation, dans le procédé selon l'invention, l'étape d'enregistrement de données physiologiques relatives au patient est une étape d'enregistrement au moins d'une pression artérielle systolique (PAS) du patient, par les moyens d'enregistrement de données physiologiques qui sont configurés pour mesurer et enregistrer au moins une pression artérielle systolique (PAS), la sous-étape de détermination d'un bolus étant réalisée par le procédé lorsque la pression artérielle systolique du patient (PAS) est supérieure ou égale à l'une au moins parmi une première valeur seuil *S*_{*PAS*1} de pression artérielle systolique et une deuxième valeur seuil *S*_{*PAS*2} de pression artérielle systolique.

Dans le dispositif d'évaluation selon l'invention, la réception de données physiologiques relatives au patient comprend la réception au moins d'une donnée de pression artérielle systolique (PAS) du patient, par exemple acquise par les moyens d'enregistrement de données physiologiques qui sont configurés pour mesurer et enregistrer au moins une pression artérielle systolique (PAS), la détermination d'un bolus étant réalisée lorsque la pression artérielle systolique du patient (PAS) est supérieure ou égale à l'une au moins parmi une première valeur seuil *S*_{*PAS*1} de pression artérielle systolique et une deuxième valeur seuil *S*_{*PAS*2} de pression artérielle systolique.

Selon un mode de réalisation, dans le procédé et le dispositif d'évaluation selon l'invention, la première valeur seuil *S*_{*PAS*1} de pression artérielle systolique correspond à une valeur moyenne prédéterminée de pression artérielle systolique, et la deuxième valeur seuil *S*_{*PAS*2} de pression artérielle systolique correspond à une pression artérielle systolique du patient mesurée et enregistrée précédemment.

En particulier, dans le procédé selon l'invention, la deuxième valeur seuil *S*_{*PAS*2} de pression artérielle systolique correspond à une pression artérielle systolique du patient mesurée et enregistrée précédemment à l'exécution dudit procédé.

Par une telle fixation des seuils, il est possible d'adapter la régulation proportionnelle en fonction d'une pression artérielle systolique moyenne généralement admise, et une pression artérielle systolique du patient précédant l'exécution du procédé.

Selon un mode de réalisation, dans le procédé selon l'invention, la pression artérielle systolique du patient mesurée et enregistrée précédemment correspond à une pression artérielle systolique du patient mesurée et enregistrée lors d'une précédente exécution dudit procédé.

Dans le dispositif d'évaluation selon l'invention, la pression artérielle systolique du patient mesurée et enregistrée précédemment correspond à une pression artérielle systolique du patient, obtenue lors d'une précédente réception des données physiologiques du patient par le dispositif d'évaluation.

Lorsque le procédé est exécuté en boucle (pas conforme à l'invention), ou que le dispositif d'évaluation évalue les besoins en boucle, la régulation proportionnelle peut tenir compte de la pression artérielle systolique mesurée et enregistrée lors d'une précédente itération du procédé ou du fonctionnement du dispositif d'évaluation, ceci permettant de suivre l'évolution de la pression artérielle systolique.

Selon un mode de réalisation, le procédé comprend une étape de régulation proportionnelle d'une quantité de composé médicamenteux à administrer, réalisée par le procédé en amont de l'étape de troisième détermination, lorsque la pression artérielle systolique du patient (PAS) est strictement inférieure à la fois à ladite première valeur seuil *S*_{*PAS*1} et à ladite deuxième valeur seuil *S*_{*PAS*2} de pression artérielle systolique, ladite étape de régulation comprenant la détermination d'une quantité de composé médicamenteux à administrer correspondant à une quantité de composé médicamenteux précédemment administré, à laquelle est soustraite une quantité de composé médicamenteux directement proportionnelle à une variation de la valeur de la pression artérielle systolique du patient (PAS) mesurée et enregistrée, par rapport à une valeur de la pression artérielle systolique du patient mesurée et enregistrée précédemment à l'exécution dudit procédé.

Dans le dispositif d'évaluation selon l'invention, les instructions comprennent la régulation proportionnelle d'une quantité de composé médicamenteux à administrer, réalisée en amont de la détermination du besoin en en composé, lorsque la pression artérielle systolique du patient (PAS) est strictement inférieure à la fois à ladite première valeur seuil *S*_{*PAS*1} et à ladite deuxième valeur seuil *S*_{*PAS*2} de pression artérielle systolique, ladite régulation comprenant la détermination d'une quantité de composé médicamenteux à administrer correspondant à une quantité de composé médicamenteux précédemment administré, à laquelle est soustraite une quantité de composé médicamenteux directement proportionnelle à une variation de la valeur de la pression artérielle systolique du patient (PAS) mesurée et enregistrée, par rapport à une valeur de la pression artérielle systolique du patient reçue lors d'une précédente évaluation par le dispositif d'évaluation.

Par une telle régulation proportionnelle à la diminution de la pression artérielle systolique, la quantité de composé à administrer peut être réduite, ce qui diminue le risque d'hypotension du patient. De plus, la quantité totale de composé à administrer peut être réduite, et davantage adaptée en fonction de l'état du patient.

Ainsi, lorsque la pression artérielle systolique du patient chute de manière trop importante, la quantité de composé médicamenteux est réduite.

Il peut s'agit ici d'une quantité de composé médicamenteux déterminée avant toute utilisation du procédé ou évaluation par le dispositif d'évaluation, ou bien d'une quantité de composé médicamenteux déterminée au cours de l'étape de troisième détermination dudit procédé, lors d'une exécution précédente du procédé, ou précédente détermination du besoin en composé par le dispositif d'évaluation.

Lorsque le procédé est exécuté en boucle, ou que le dispositif d'évaluation évalue en boucle, on comprend que la détermination du besoin en composé, et donc la détermination d'un bolus, n'est réalisée que lorsque la régulation, exécutée un nombre suffisant de fois, a permis de vérifier la condition selon laquelle la pression artérielle systolique n'est plus inférieure à l'un des deux seuils de pression artérielle systolique précités.

Ceci permet de sécuriser le procédé ou l'utilisation du dispositif d'évaluation, en assurant qu'aucune quantité de composé trop importante, et en particulier aucun bolus, ne risque d'être administrée au patient qui serait en situation d'hypotension.

Selon un troisième aspect, l'invention a également pour objet un dispositif de délivrance d'un composé médicamenteux, comprenant un dispositif d'évaluation des besoins en composé médicamenteux d'un patient selon un deuxième aspect de l'invention, associé à des moyens de commande et à un système de perfusion, de préférence électrique.

Le dispositif de délivrance peut notamment comprendre des moyens de commande configurés pour réguler un débit de composé médicamenteux en fonction de la quantité de composé médicamenteux déterminé, par l'intermédiaire d'une action sur le système de perfusion.

### BRÈVE DESCRIPTION DES FIGURES

D'autres avantages, buts et caractéristiques particulières de la présente invention ressortiront de la description non limitative qui suit d'au moins un mode de réalisation particulier des dispositifs et procédés objets de la présente invention, en regard des dessins annexés, dans lesquels :
- la figure 1 représente schématiquement un dispositif de délivrance comprenant un dispositif d'évaluation selon l'invention ;
- la figure 2 représente schématiquement un dispositif d'évaluation selon l'invention ;
- la figure 3 est un logigramme du procédé d'évaluation selon l'invention ;
- la figure 4 est un logigramme du procédé d'évaluation selon l'invention, comprenant une étape optionnelle de régulation proportionnelle ;
- la figure 5 représente les graphe de la fonction d'appartenance F_MC de l'indice MC et le graphe de la fonction d'appartenance F_PC de l'indice PC ;
- la figure 6 est un logigramme du procédé d'évaluation, selon un mode de réalisation.

### DESCRIPTION DÉTAILLÉE DE L'INVENTION

La présente description est donnée à titre non limitatif, chaque caractéristique d'un mode de réalisation pouvant être combinée à toute autre caractéristique de tout autre mode de réalisation de manière avantageuse.

On note, dès à présent, que les figures ne sont pas à l'échelle.

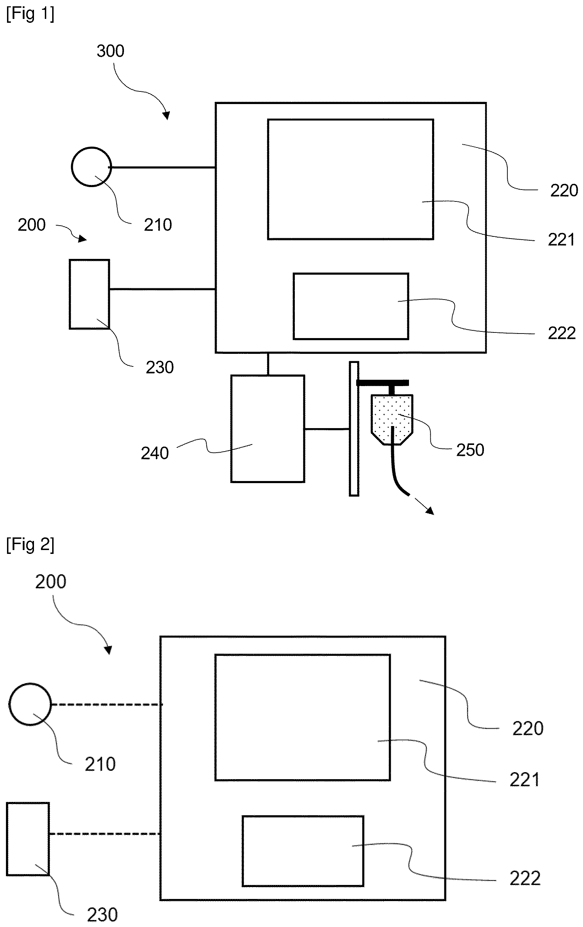

La figure 1 représente schématiquement un dispositif de délivrance 300 de composé.

Le dispositif de délivrance 300 comprend un dispositif d'évaluation 200 des besoins en composé médicamenteux, des moyens de commande 240 et un système de perfusion 250.

De préférence, le système de perfusion 250 est électrique, et est actionné par les moyens de commande 240, reliés par une liaison de données au dispositif d'évaluation 200. Le système de perfusion est adapté pour distribuer un composé médicamenteux dans le circuit de circulations sanguine d'un patient nécessitant l'administration du composé médicamenteux, par exemple via un cathéter du système de perfusion 250.

Le composé médicamenteux est en particulier un composé analgésique et/ou hypnotique.

En particulier, le composé analgésique et/ou hypnotique est un composé analgésique, par exemple un composé opioïde, notamment morphinique. Par exemple, le composé est du remifentanil. Selon d'autres exemples, le composé est un composé non-morphinique tel que de la kétamine, ou un composé de la classe des alpha-2-agonistes, tel que la clonidine.

La figure 2 représente schématiquement un dispositif d'évaluation 200 seul, c'est-à-dire non associé notamment à des moyens de commande 240 et un système de perfusion 250. Le dispositif d'évaluation 200 peut en effet fonctionner seul sans moyens d'administration du composé, en déterminant les besoins en composé analgésique et/ou hypnotique et/ou curarisant et en transmettant cette information à un autre dispositif, ou à un personnel médical par exemple.

Le dispositif d'évaluation 200 peut notamment comprendre des moyens de mesure 210 de la variabilité cardiaque du patient entre 0.15Hz et 4Hz, une unité de traitement et d'enregistrement de données 220 et des moyens d'enregistrement 230 de données physiologiques relatives au patient.

Par exemple, l'unité de traitement et d'enregistrement de données 220 comprend un micro-processeur, ou processeur, 221 et une mémoire informatique 222, adaptée pour stocker des instructions configurant le processeur 221 pour son fonctionnement. Par exemple, la mémoire informatique 222 est une mémoire informatique de stockage non volatile, pouvant être réinscriptible, étant par exemple de type « EPROM ».

Les moyens d'enregistrement 230 de données physiologiques relatives au patient peuvent être des capteurs de données physiologiques, notamment des capteurs disponibles dans le commerce. Ces capteurs peuvent être configurés pour enregistrer des données physiologiques, ou vitales, du patient, et les transmettre par une liaison de données à l'unité de traitement et d'enregistrement de données 220. Par exemple, les moyens d'enregistrement 230 comprennent au moins un capteur de pression artérielle, notamment systolique, également appelé tensiomètre.

Les moyens de mesure 210 de la variabilité cardiaque du patient entre 0.15Hz et 4Hz peuvent comprendre différents moyens connus de l'homme du métier, tel qu'un électrocardiographe (ECG) et/ou un oxymètre, par l'intermédiaire du ou desquels la variabilité cardiaque du patient entre 0.15Hz et 4Hz peut être déterminée.

Le dispositif d'évaluation 200 peut également comprendre d'autres périphériques, tels que des moyens d'affichage d'informations, par exemple un écran informatique.

Le dispositif d'évaluation 200 permet d'évaluer, à partir de données physiologiques du patient, d'évaluer un besoin en composé analgésique et/ou hypnotique d'un patient, ci-après appelés « composé », en particulier lorsque le patient est placé sous anesthésie ou sédation.

Le besoin en composé peut notamment être la détermination d'une quantité de composé nécessitée par le patient. Le dispositif d'évaluation 200 peut permettre de communiquer une telle quantité de composé à un personnel médical, par exemple via un écran informatique, ou bien communiquer la quantité de composé aux moyens de commande 240 en vue d'une administration par le système de perfusion 250.

Ainsi, le dispositif d'évaluation 200 permet d'apporter une recommandation sur les besoins en composé d'un patient, la quantité adéquate de composé pouvant ensuite être administrée par le personnel médical, ou automatiquement par le système de perfusion, sur décision du personnel médical.

Le besoin en composés est évalué par le dispositif d'évaluation 200 notamment à partir d'un indice ANI, qui est un indice relatif au niveau de douleur ressenti par le patient. Ainsi, le besoin en composé peut être déterminé en fonction d'un niveau de douleur ressenti, et ainsi évaluer la quantité optimale de composé nécessaire au patient pour atténuer la douleur, sans sous-doser ou sur-doser la quantité de composé.

A cet effet, le dispositif d'évaluation 200 est configuré pour mettre en œuvre un procédé 100 d'évaluation des besoins en composé analgésique et/ou hypnotique d'un patient.

Plus précisément, la mémoire informatique 222 du dispositif d'évaluation 200 stocke des instructions configurant le processeur 221 pour mettre en œuvre les étapes du procédé 100.

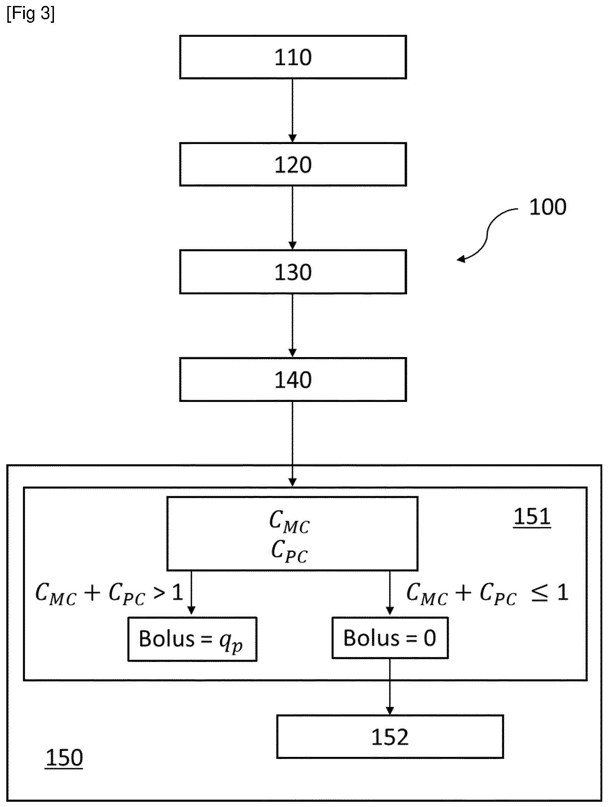

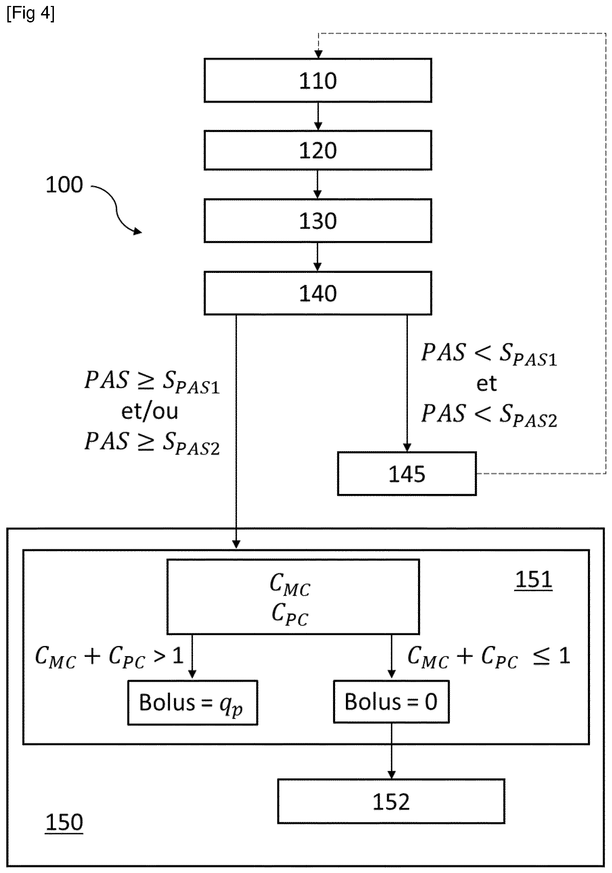

La procédé 100, destiné à être mis en œuvre par le dispositif d'évaluation 200, va maintenant être décrit, en référence aux figures 3 et 4 notamment.

Le procédé 100 comprend une étape de mesure 110 de la variabilité cardiaque du patient entre 0.15Hz et 4Hz.

Le procédé 100 comprend ensuite une étape de première détermination 120 par l'unité de traitement et d'enregistrement de données 220, à partir des données de variabilité cardiaque mesurées lors de l'étape 110, d'un indice ANI relatif au niveau de douleur du patient.

Par exemple, l'indice ANI peut être calculé selon le procédé décrit dans la demande WO 2006/032739. L'étape de première détermination 120 peut ainsi comprendre les étapes d'un tel procédé afin de déterminer l'indice ANI.

Le procédé 100 comprend en outre une étape de deuxième détermination 130 par l'unité de traitement et d'enregistrement de données 220 d'un sous-indice, ou indice, MC et d'un sous-indice, ou indice, ML.

Les indices MC et ML sont déterminés à partir de l'indice ANI, l'indice MC étant représentatif de la moyenne de l'indice ANI sur une période dite courte, et l'indice ML étant représentatif de la moyenne de l'indice ANI sur une période dite longue.

Les indices MC et ML correspondent donc au niveau de douleur d'un patient, respectivement sur une période dite courte et une période dite longue, de durée supérieure à la période courte.

En outre, dans l'étape de deuxième détermination 130, sont déterminées les pentes PC et PL respectivement desdits indices MC et ML, correspondant à l'évolution du niveau de douleur d'un patient, respectivement sur la période courte et la période longue.

En d'autres termes, les indices MC et ML sont calculés à partir des différentes valeurs de l'ANI pendant une durée donnée, et permettent de limiter l'impact de la variabilité instantanée de la mesure de l'ANI due à la méthode de calcul et également de dégager des tendances dans l'évolution de la douleur du patient.

Plus précisément, le calcul des indices MC et ML est de préférence réalisé à partir d'une moyenne des valeurs des indices ANI sur deux périodes différentes à savoir une période courte et une période longue.

De manière avantageuse, la période courte correspond à une fenêtre glissante de 45 à 90 secondes et de préférence de l'ordre de 60 secondes, tandis que la période longue correspond à une fenêtre glissante de 90 à 240 secondes et de préférence de l'ordre de 180 secondes.

De manière avantageuse, la période prise en compte pour le calcul de ML est sensiblement de l'ordre de 1.5 à 3 fois la période utilisée pour le calcul de MC.

La pente PC est calculée en degrés à partir de deux valeurs MC séparés d'une durée donnée selon la formule suivante : $PC = \left(\frac{180}{π}\right) arctan \left(\frac{MC \left(t2\right) - MC \left(t1\right)}{t 2 - t 1}\right)$.

Avantageusement l'intervalle de temps entre t1 et t2 est de l'ordre de 15 à 45 secondes et de préférence égal à 30 secondes.

La pente PL est calculée en degrés de manière analogue à celle de PC selon la formule suivante : $PL = \left(\frac{180}{π}\right) arctan \left(\frac{ML \left(t2\right) - ML \left(t1\right)}{t 2 - t 1}\right)$.

Là encore l'intervalle de temps séparant t1 et t2 est de l'ordre de 15 à 45 secondes et de préférence égal à 30 secondes.

De plus, le procédé 100 comprend une étape d'enregistrement 140 de données physiologiques relatives au patient, par les moyens d'enregistrement 230 de données physiologiques relatives au patient.

Comme cela a été présenté plus en amont, les moyens d'enregistrement 230 permettent par exemple la mesure de la fréquence cardiaque (FC) et de la pression artérielle systolique (PAS).

Ces paramètres ne sont pas utilisés directement dans le calcul de la quantité de composés optimale que nécessite le patient, mais sont utilisés de sorte à constituer des sécurités, le dispositif d'évaluation ne réalisant pas d'évaluation lorsque certains seuils correspondant à ces paramètres sont dépassés.

Un exemple d'étape de sécurité sera décrit dans la suite de la présente description.

Dans d'autres modes de réalisation, d'autres paramètres physiologiques pourraient être utilisés en remplacement ou additionnellement à la fréquence cardiaque (FC) et à la pression artérielle systolique (PAS).

Il est également envisageable, bien que ce mode présenterait l'inconvénient d'une sécurité dégradée, de n'utiliser qu'un seul paramètre physiologique.

Il est aussi envisageable d'enregistrer par les moyens d'enregistrement 230 d'autres types de données relatives au patient tels que le poids, la taille, l'âge ou encore le sexe du patient. A cet effet les moyens d'enregistrement 230 peuvent comprendre avantageusement un clavier ou surface tactile, non représenté dans les figures annexées.

Le procédé 100 comprend également une étape de troisième détermination 150 par l'unité de traitement et d'enregistrement de données 220, du besoin en composé médicamenteux, ici par exemple en composé analgésique et/ou hypnotique, nécessité par le patient

Le besoin en composé analgésique et/ou hypnotique nécessité par le patient est déterminé en fonction des indices MC et ML, de leurs pentes PC et PL, et des données physiologiques relatives au patient.

Plus précisément, au cours de l'étape de troisième détermination 150 il est d'abord déterminé si le patient nécessite l'administration d'une première quantité *qₚ* dudit composé, à administrer ponctuellement au patient. Une telle quantité relativement importante de composé, et destinée à être administrée sur un temps court, est appelée « bolus ». Le patient nécessite l'administration d'un bolus de composé lorsque la douleur ressentie est aiguë, c'est-à-dire lorsque l'indice ANI est proche de 0.

A cet effet, l'étape de troisième détermination 150 comprend une sous-étape 151 de détermination d'une première quantité du composé à administrer ponctuellement au patient, c'est-à-dire une sous-étape 151 de détermination d'un bolus.

La sous-étape 151 de détermination d'un bolus comprend la détermination d'une première variable de logique floue *C_{MC}*, comprise entre 0 et 1, à partir d'une fonction d'appartenance *F_{MC}* de l'indice MC.

La sous-étape 151 de détermination d'un bolus comprend également la détermination d'une deuxième variable de logique floue *C_{PC}* , comprise entre 0 et 1, à partir d'une fonction d'appartenance *F_{PC}* de l'indice PC.

La sous-étape 151 de détermination d'un bolus comprend ensuite la détermination que le bolus est égal à une première quantité prédéterminée *qₚ* dudit composé lorsque la somme de la première variable de logique floue *C_{MC}* et de la deuxième variable de logique floue *C_{PC}* est strictement supérieure à 1, et que le bolus est égal à zéro sinon.

En d'autres termes, le patient nécessite un bolus lorsque la somme de la première variable de logique floue *C_{MC}* et de la deuxième variable de logique floue *C_{PC}* est strictement supérieure à 1, et ne nécessite pas de bolus lorsque la somme de la première variable de logique floue *C_{MC}* et de la deuxième variable de logique floue *C_{PC}* est inférieure à 1.

La sous-étape 151 de détermination d'un bolus est ainsi une étape faisant appel à une logique floue pour déterminer la nécessité d'administrer ou non un bolus.

Les fonction d'appartenance *F_{MC}* de l'indice MC et fonction d'appartenance *F_{PC}* de l'indice PC sont ici des fonctions bornées entre 0 et 1. Ainsi, l'image d'une valeur d'indice MC ou PC par la fonction d'appartenance respective est une valeur *C_{PC}* ou *C_{MC}* comprise entre 0 et 1, c'est-à-dire une valeur booléenne « floue ».

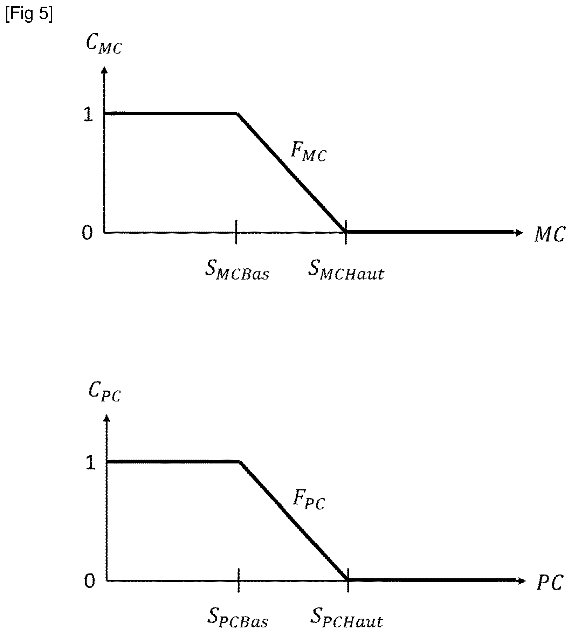

La figure 5 représente les graphes des fonction d'appartenance *F_{MC}* de l'indice MC et fonction d'appartenance *F_{PC}* de l'indice PC.

Par exemple, la fonction d'appartenance *F_{MC}* de l'indice MC est :
- Egale à 1 lorsque l'indice MC est inférieur ou égal à une valeur seuil basse *S_{MCBas}* ;
- Egale à 0 lorsque l'indice MC est supérieur ou égal à une valeur seuil haute *S_{MCHaut} ;*
- Et présente une transition décroissante monotone entre 1 et 0, lorsque l'indice MC est compris entre les valeurs seuil basse *S_{MCBas}* et haute *S_{MCHaut}*,

De manière similaire, par exemple, la fonction d'appartenance *F_{PC}* de l'indice PC est :
- Egale à 1 lorsque l'indice PC est inférieur ou égal à une valeur seuil basse *S_{PCBas}* ;
- Egale à 0 lorsque l'indice PC est supérieur ou égal à une valeur seuil haute *S_{PCHaut}* ;
- Et présente une transition décroissante monotone entre 1 et 0, lorsque l'indice PC est compris entre les valeurs seuil basse *S_{PCBas}* et haute *S_{PCHaut}*.

Des exemples de transition décroissante monotone sont une transition linéaire, hyperbolique, notamment sigmoïde, exponentielle ou encore gaussienne.

Une transition décroissante linéaire est particulièrement préférée.

Par exemple, la fonction d'appartenance *F_{MC}* de l'indice MC est définie selon l'expression : ${F}_{MC} = \left\{\begin{matrix}1 , & si MC \leq {S}_{MCBas} \\ \frac{MC - {S}_{MCHaut}}{{S}_{MCBas} - {S}_{MCHaut}} , & si {S}_{MCBas} < MC < {S}_{MCHaut} \\ 0 , & si MC \geq {S}_{MCHaut}\end{matrix}\right.$.

Par exemple, la fonction d'appartenance *F_{PC}* de l'indice PC est définie selon l'expression : ${F}_{PC} = \left\{\begin{matrix}1 , & si PC \leq {S}_{PCBas} \\ \frac{PC - {S}_{PCHaut}}{{S}_{PCBas} - {S}_{PCHaut}} , & si {S}_{PCBas} < PC < {S}_{PCHaut .} \\ 0 , & si PC \geq {S}_{PCHaut}\end{matrix}\right.$.

Les seuils de transition hauts et bas *S_{MCBas}*, *S_{MCHaut}*, *S_{PCBas}* et *S_{PCHaut}* peuvent être adaptés librement selon les conditions d'utilisation du procédé 100, notamment en fonction du profil du patient (notamment son âge, poids, sexe, etc.), et/ou le type de composé destiné à être administré au patient, et/ou la condition clinique du patient, c'est-à-dire selon ses besoins en soins médicaux. De même, le type de transition peut être adapté librement selon ces mêmes critères.

Par exemple, les valeurs des seuils *S_{MCBas}*, *S_{MCHaut}*, *S_{PCBas}* et *S_{PCHaut}* peuvent être fixés comme suit :
- La valeur seuil *S_{MCBas}* est comprise entre 45 et 55, et préférentiellement égale à 50.
- La valeur seuil *S_{MCHaut}*est comprise entre 58 et 68, et préférentiellement égale à 63.
- La valeur seuil *S_{PCBas}* est comprise entre -40 et -30 et préférentiellement égale à -35.
- La valeur seuil *S_{PCHaut}* est comprise entre -30 et -20, et préférentiellement égale à -25.

De telles plages de valeurs, et valeurs préférentielles, notamment associées à une transition linéaire, sont particulièrement adaptées lors d'une chirurgie sous anesthésie générale, lorsque du remifentanil en tant que composé doit être administré au patient. En effet, un fonctionnement répétible et stable du procédé a pu être observé dans ce contexte.

La sous-étape 151 de détermination d'un bolus, basée sur une logique floue, permet d'obtenir la détermination d'un bolus particulièrement fiable et répétible, même lorsque l'indice MC présente une forte variabilité, en particulier lorsqu'il est déterminé sur une fenêtre glissante. En effet, la logique floue permet de s'affranchir d'une détermination de bolus basée sur des seuils fixes, qui est beaucoup plus sensible aux variations de l'indice MC.

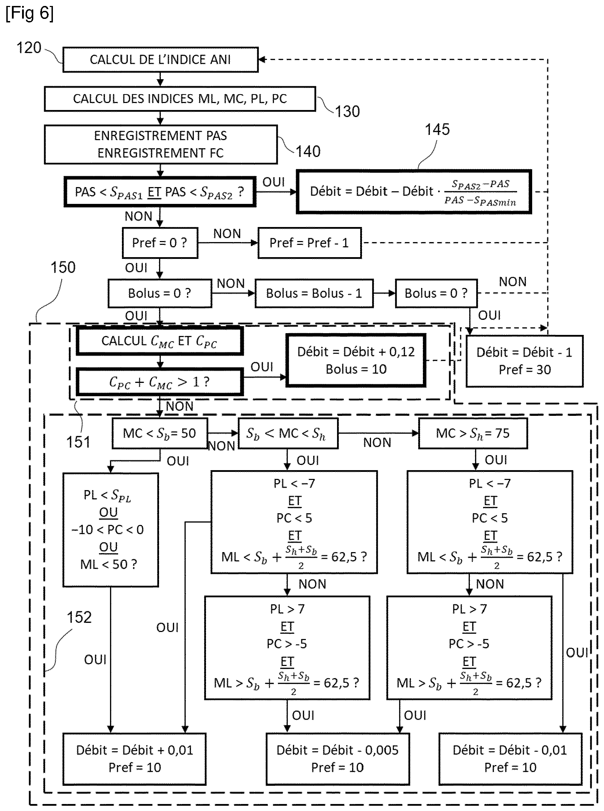

De manière concrète, en référence à la figure 6 qui montre un exemple plus détaillé d'implémentation du procédé 100 sous forme d'un logigramme, lorsqu'un bolus est déterminé, et lorsque le composé utilisé est du remifentanil de concentration à 25µg/ml, une variable de débit est par exemple augmentée en fonction du poids du patient de 0.12 µg/kg/mn pendant 10 secondes.

Dans cet exemple, une variable « Bolus » est fixée à 10, cette variable étant décrémentée lors d'une prochaine exécution du procédé 100, qui peut fonctionner en boucle en continu ou périodiquement. Tant que la variable « Bolus » n'est pas nulle, aucune nouvelle étape de détermination d'une quantité de composé n'est réalisée par le procédé 100.

Si, à l'issue de la sous-étape 151 de détermination d'un bolus, la somme de la première variable de logique floue *C_{MC}* et de la deuxième variable de logique floue *C_{PC}* est inférieure à 1, c'est-à-dire que le bolus est nul, le procédé 100 exécute une sous-étape 152 de détermination d'une deuxième quantité du composé à administrer, inférieure à ladite première quantité prédéterminée *qₚ*.

La deuxième quantité du composé est déterminée en fonction des indices MC et ML, de leurs pentes PC et PL, et des données physiologiques relatives au patient.

Par exemple, la sous-étape 152 de détermination d'une deuxième quantité du composé comprend une série de comparaison successives des indices MC, ML, PC et PL à des seuils prédéterminés.

En particulier, tel que cela est visible à la figure 6 au niveau de la sous-étape 152, l'indice MC est d'abord comparé successivement à des seuils bas *S_{b}* et haut *Sₕ*, afin de déterminer si la moyenne de l'indice ANI sur une période courte est basse, moyenne, ou haute, c'est-à-dire si la douleur est forte, modérée, ou faible.

En fonction de cette première détermination, en fonction de la tendance sur une période longue du niveau de douleur moyen, et de l'évolution sur les périodes courtes et longue du niveau de douleur de moyen, le procédé 100 détermine la deuxième quantité de composé. La deuxième quantité de composé peut être plus importante ou plus faible qu'une deuxième quantité de composé déterminée précédemment, notamment déterminée lors d'une précédente exécution du procédé 100. Il est également possible que la deuxième quantité de composé demeure inchangée par rapport à une deuxième quantité de composé déterminée précédemment.

Une fois la sous-étape 152 exécutée, et la deuxième quantité de composé déterminée, le procédé 100 peut être exécuté à nouveau depuis le début. Il est possible de temporiser une nouvelle exécution du procédé, par exemple en fixant une valeur de la variable « Pref », ici à 10 sur l'exemple de la figure 6, cette variable étant décrémentée lors de chaque exécution du procédé, avant l'exécution de l'étape de troisième détermination 150.

La mise en œuvre spécifique de la sous-étape 152, ainsi que le fonctionnement spécifique des boucles de décrémentation des variables « Bolus » et « Pref » de l'algorithme illustré à la figure 6, sont décrites dans les documents de brevet FR 3006879 et FR 3006880 du Demandeur et auquel l'homme du métier pourra se référer pour la mise en œuvre du procédé 100.

Tel que cela a été évoqué plus en amont de la présente description, le procédé 100 peut comprendre une étape de sécurité, visant à empêcher toute détermination de quantité de composé à administrer lorsque certains seuils de données physiologiques du patient sont dépassés.

Par exemple, en référence aux figures 4 et 6, le procédé 100 comprend une étape optionnelle de comparaison de la pression artérielle systolique (PAS) à deux seuils de pression artérielle systolique.

A cet effet, l'étape d'enregistrement 140 de données physiologiques relatives au patient est alors une étape d'enregistrement au moins d'une pression artérielle systolique (PAS) du patient, par les moyens d'enregistrement 230 de données physiologiques qui sont alors configurés pour mesurer et enregistrer au moins une pression artérielle systolique (PAS).

L'étape optionnelle de comparaison de la pression artérielle systolique (PAS) à deux seuils de pression artérielle systolique comprend la comparaison de la pression artérielle systolique (PAS) à :
- Un premier seuil *S*_{*PAS*1} de pression artérielle systolique qui peut être une valeur moyenne prédéterminée de pression artérielle systolique, par exemple une pression artérielle systolique de 120 mmHg qui peut être considérée comme un seuil minimal de pression artérielle systolique d'une personne en bonne santé, et à :
- Un deuxième seuil *S*_{*PAS*2} de pression artérielle systolique qui peut être une valeur de pression artérielle systolique (PAS) du patient, mesurée et enregistrée précédemment à l'exécution du procédé 100. Par exemple, la pression artérielle systolique (PAS) du patient peut avoir été mesurée et enregistrée lors d'une exécution précédente du procédé 100.

Lorsque la pression artérielle systolique (PAS) du patient est supérieure ou égale à l'une au moins parmi la première valeur seuil *S*_{*PAS*1} de pression artérielle systolique et la deuxième valeur seuil *S*_{*PAS*2} de pression artérielle systolique, la sous-étape 151 de détermination d'un bolus est exécutée par le procédé 100.

En effet, lorsque la pression artérielle systolique (PAS) du patient n'est pas inférieure au premier seuil, et ne baisse pas par rapport à une pression artérielle systolique précédente, il est possible de considérer que le risque d'hypotension du patient est faible, et qu'une quantité de composé à administrer peut être déterminée sans danger pour la santé du patient.

Lorsque la pression artérielle systolique du patient (PAS) est strictement inférieure à la fois à la première valeur seuil *S*_{*PAS*1} et à la deuxième valeur seuil *S*_{*PAS*2} de pression artérielle systolique, une étape 145 de régulation proportionnelle d'une quantité de composé analgésique et/ou hypnotique à administrer est réalisée par le procédé en amont de l'étape de troisième détermination 150. L'étape de troisième détermination 150, et ainsi notamment la détermination d'un bolus, n'est alors pas exécutée par le procédé 100 au cours de la même itération du procédé 100.

L'étape 145 de régulation proportionnelle comprend la détermination d'une quantité de composé analgésique et/ou hypnotique à administrer, cette quantité correspondant à une quantité de composé analgésique et/ou hypnotique précédemment administré, à laquelle est soustraite une quantité de composé analgésique et/ou hypnotique directement proportionnelle à une variation de la valeur de la pression artérielle systolique du patient (PAS) mesurée et enregistrée, par rapport à une valeur de la pression artérielle systolique du patient mesurée et enregistrée précédemment à l'exécution du procédé 100.

Concrètement, en référence à l'algorithme illustré à la figure 6, une variable « Débit » de consigne de débit de composé peut être mise à jour de manière directement proportionnelle à la variation de la valeur de la pression artérielle systolique du patient (PAS) mesurée et enregistrée, par rapport à une valeur de la pression artérielle systolique du patient mesurée et enregistrée précédemment à l'exécution du procédé 100.

Par exemple, une nouvelle consigne de débit peut être définie de la manière suivante : $Débit = Débit - Débit ⋅ \frac{{S}_{PAS 2} - PAS}{PAS - {S}_{PASmin}}$, où *S_{PASmin}* est une valeur seuil prédéterminée de pression artérielle systolique, par exemple correspondant à 80 mmHg.

En d'autres termes, la variable « Débit » est écrasée et mise à jour de manière à réduire la consigne de débit de composé de manière proportionnelle à l'évolution de la pression artérielle systolique du patient (PAS).

L'étape 145 de régulation proportionnelle est exécutée en boucle jusqu'à ce que la pression artérielle systolique du patient ne soit plus inférieure à l'une des deux valeurs seuil *S*_{*PAS*1} et *S*_{*PAS*2}, c'est-à-dire que le risque d'hypotension du patient peut être considéré comme faible. Ceci constitue une mesure de sécurité, évitant d'évaluer des besoins en composé trop importants pour un patient en situation d'hypotension, ou de chute de tension.

Par exemple, l'intervalle entre deux itérations, c'est-à-dire entre deux mesures de pression artérielle systolique du patient, est compris entre 2 et 5 minutes.

De plus, l'étape 145 de régulation proportionnelle permet une meilleure adaptation de l'administration du composé en fonction des besoins du patient, et permet également de diminuer la quantité totale de composé devant être administré au patient, ce qui est avantageux à la fois sur le plan clinique et le plan économique.

Bien que l'étape optionnelle de comparaison de la pression artérielle systolique (PAS) à deux seuils de pression artérielle systolique, suivie le cas échéant d'une étape 145 de régulation proportionnelle, soit particulièrement avantageuse tel que présenté ci-dessus, il est également envisageable de prévoir une étape de sécurité plus classique telle que décrite dans les documents de brevet FR 3006879 et FR 3006880 du Demandeur. L'étape de sécurité qui y est décrite prévoit un arrêt du débit de composé lorsque le patient est en hypotension ou en bradycardie.

Selon un mode de réalisation, le procédé 100, et/ou le dispositif d'évaluation 200, et/ou le dispositif de délivrance 300 peuvent être destinés à l'évaluation d'un besoin en soins paramédicaux d'un patient, de façon alternative ou complémentaire à l'évaluation du besoin en composé médicamenteux.

En d'autres termes, en complément ou en alternative de la détermination d'une quantité de composé que nécessite le patient, il est possible d'évaluer de manière similaire, c'est-à-dire sur la base d'une logique floue reposant sur un indice ANI et de ses sous-indices, les besoins en soins paramédicaux d'un patient. Par exemple, la détermination d'un besoin en soins paramédicaux comprend la détermination qu'un soin paramédical est nécessité par le patient, et peut par exemple comprendre la détermination de la durée et/ou de l'intensité du soin paramédical.

On entend par « soin paramédical » l'ensemble des soins pouvant être apportés à un patient à l'exception des traitements médicaux et chirurgicaux, il s'agit notamment, de manière non limitative, des soins en pédiatrie, de kinésithérapie, de néonatologie, de neurophysiologie, ou encore de manière plus large de soins basés sur une modification de l'environnement de sorte à améliorer le bien-être du patient.

On rappelle plus généralement que l'invention ne se limite pas aux exemples décrits et illustrés.

## Revendications

1. Procédé (100) d'évaluation des besoins en composé médicamenteux d'un patient, destiné à être mis en œuvre par un dispositif d'évaluation (200) comprenant des moyens de mesure (210) de la variabilité cardiaque du patient entre 0.15Hz et 4Hz, une unité de traitement et d'enregistrement de données (220), et des moyens d'enregistrement (230) de données physiologiques relatives au patient, le procédé comprenant les étapes suivantes :
• une étape de mesure (110) de la variabilité cardiaque du patient entre 0.15Hz et 4Hz ;
• une étape de première détermination (120) par l'unité de traitement et d'enregistrement de données (220), à partir des données de variabilité cardiaque mesurées, d'un indice ANI relatif au niveau de douleur du patient ;
• une étape de deuxième détermination (130) par l'unité de traitement et d'enregistrement de données (220), à partir de l'indice ANI, d'un indice MC, représentatif de la moyenne de l'indice ANI sur une période dite courte, et d'un indice ML, représentatif de la moyenne de l'indice ANI sur une période dite longue, ainsi que les pentes PC et PL respectivement desdits indices MC et ML, les indices MC et ML correspondant au niveau de douleur d'un patient et les indices PC et PL à son évolution, respectivement sur une période courte et une période longue ;
• une étape d'enregistrement (140) de données physiologiques relatives au patient, par les moyens d'enregistrement (230) de données physiologiques relatives au patient ;
• une étape de troisième détermination (150) par l'unité de traitement et d'enregistrement de données (220), en fonction des indices MC et ML, de leurs pentes PC et PL, et des données physiologiques relatives au patient, le besoin en composé médicamenteux nécessité par le patient, ladite étape de troisième détermination comprenant une sous-étape (151) de détermination d'une première quantité dudit composé à administrer ponctuellement au patient, dit bolus, comprenant :
∘ la détermination d'une première variable de logique floue *C_{MC}*, comprise entre 0 et 1, à partir d'une fonction d'appartenance *F_{MC}* de l'indice MC,
∘ la détermination d'une deuxième variable de logique floue *C_{PC}*, comprise entre 0 et 1, à partir d'une fonction d'appartenance *F_{PC}* de l'indice PC,
∘ la détermination que le bolus est égal à une première quantité prédéterminée dudit composé lorsque la somme de la première variable de logique floue *C_{MC}* et de la deuxième variable de logique floue *C_{PC}* est strictement supérieure à 1, et que le bolus est égal à zéro sinon ;
ladite étape de troisième détermination (150) comprenant en outre une sous-étape (152) de détermination d'une deuxième quantité dudit composé à administrer, inférieure à ladite première quantité prédéterminée, déterminée en fonction des indices MC et ML, de leurs pentes PC et PL, et des données physiologiques relatives au patient, cette sous-étape (152) étant exécutée par le procédé à la suite de la sous-étape (151) de détermination d'un bolus lorsque le bolus est égal à zéro.

2. Procédé (100) selon la revendication 1, dans lequel la fonction d'appartenance *F_{MC}* de l'indice MC est :
• égale à 1 lorsque l'indice MC est inférieur ou égal à une valeur seuil basse *S_{MCBas}*,
• égale à 0 lorsque l'indice MC est supérieur ou égal à une valeur seuil haute *S_{MCHaut}*,
et présente une transition décroissante monotone entre 1 et 0, lorsque l'indice MC est compris entre les valeurs seuil basse *S_{MCBas}* et haute *S_{MCHaut}*, et dans lequel fonction d'appartenance *F_{PC}* de l'indice PC est :
• égale à 1 lorsque l'indice PC est inférieur ou égal à une valeur seuil basse *S_{PCBas}*,
• égale à 0 lorsque l'indice PC est supérieur ou égal à une valeur seuil haute *S_{PCHaut}*,
et présente une transition décroissante monotone entre 1 et 0, lorsque l'indice PC est compris entre les valeurs seuil basse *S_{PCBas}* et haute *S_{PCHaut}*.

3. Procédé (100) selon la revendication 2, dans lequel la fonction d'appartenance *F_{MC}* de l'indice MC est définie selon l'expression : ${F}_{MC} = \left\{\begin{matrix}1 , & si MC \leq {S}_{MCBas} \\ \frac{MC - {S}_{MCHaut}}{{S}_{MCBas} - {S}_{MCHaut}} , & si {S}_{MCBas} < MC < {S}_{MCHaut} \\ 0 , & si MC \geq {S}_{MCHaut}\end{matrix}\right.$ et la fonction d'appartenance *F_{PC}* de l'indice PC est définie selon l'expression : ${F}_{PC} = \left\{\begin{matrix}1 , & si PC \leq {S}_{PCBas} \\ \frac{PC - {S}_{PCHaut}}{{S}_{PCBas} - {S}_{PCHaut}} , & si {S}_{PCBas} < PC < {S}_{PCHaut .} \\ 0 , & si PC \geq {S}_{PCHaut}\end{matrix}\right.$

4. Procédé (100) selon l'une quelconque des revendications 2 ou 3, dans lequel :
• la valeur seuil *S_{MCBas}* est comprise entre 45 et 55, et préférentiellement égale à 50,
• la valeur seuil *S_{MCHaut}*est comprise entre 58 et 68, et préférentiellement égale à 63,
• la valeur seuil *S_{PCBas}* est comprise entre -40 et -30 et préférentiellement égale à -35,
• la valeur seuil *S_{PCHaut}* est comprise entre -30 et -20, et préférentiellement égale à -25.

5. Procédé (100) selon l'une quelconque des revendications 1 à 4, dans lequel l'étape d'enregistrement (140) de données physiologiques relatives au patient est une étape d'enregistrement au moins d'une pression artérielle systolique (PAS) du patient, par les moyens d'enregistrement (230) de données physiologiques qui sont configurés pour mesurer et enregistrer au moins une pression artérielle systolique (PAS), la sous-étape (151) de détermination d'un bolus étant réalisée par le procédé lorsque la pression artérielle systolique du patient (PAS) est supérieure ou égale à l'une au moins parmi une première valeur seuil *S*_{*PAS*1} de pression artérielle systolique et une deuxième valeur seuil *S*_{*PAS*2} de pression artérielle systolique.

6. Procédé (100) selon la revendication 5, dans lequel la première valeur seuil *S*_{*PAS*1} de pression artérielle systolique correspond à une valeur moyenne prédéterminée de pression artérielle systolique, et la deuxième valeur seuil *S*_{*PAS*2} de pression artérielle systolique correspond à une pression artérielle systolique du patient mesurée et enregistrée précédemment à l'exécution dudit procédé.

7. Procédé (100) selon la revendication 6, dans lequel la pression artérielle systolique du patient mesurée et enregistrée précédemment correspond à une pression artérielle systolique du patient mesurée et enregistrée lors d'une précédente exécution dudit procédé.

8. Procédé (100) selon l'une quelconque des revendications 5 à 7, comprenant en outre une étape de régulation proportionnelle (145) d'une quantité de composé médicamenteux à administrer, réalisée par le procédé en amont de la étape de troisième détermination (150), lorsque la pression artérielle systolique du patient (PAS) est strictement inférieure à la fois à ladite première valeur seuil *S*_{*PAS*1} et à ladite deuxième valeur seuil *S*_{*PAS*2} de pression artérielle systolique, ladite étape de régulation (145) comprenant la détermination d'une quantité de composé médicamenteux à administrer correspondant à une quantité de composé médicamenteux précédemment administré, à laquelle est soustraite une quantité de composé médicamenteux directement proportionnelle à une variation de la valeur de la pression artérielle systolique du patient (PAS) mesurée et enregistrée, par rapport à une valeur de la pression artérielle systolique du patient mesurée et enregistrée précédemment à l'exécution dudit procédé.

9. Dispositif d'évaluation (200) des besoins en composé médicamenteux d'un patient, comprenant des moyens de mesure (210) de la variabilité cardiaque du patient entre 0.15Hz et 4Hz, une unité de traitement et d'enregistrement de données (220), et des moyens d'enregistrement (230) de données physiologiques relatives au patient, l'unité de traitement et d'enregistrement comprenant un processeur (221) et une mémoire informatique (222) stockant des instructions configurant le processeur pour :
• recevoir des données de la variabilité cardiaque du patient entre 0.15Hz et 4Hz ;
• déterminer, à partir des données de variabilité cardiaque reçues, d'un indice ANI relatif au niveau de douleur du patient ;
• déterminer, à partir de l'indice ANI, d'un indice MC, représentatif de la moyenne de l'indice ANI sur une période dite courte, et d'un indice ML, représentatif de la moyenne de l'indice ANI sur une période dite longue, ainsi que les pentes PC et PL respectivement desdits indices MC et ML, les indices MC et ML correspondant au niveau de douleur d'un patient et les indices PC et PL à son évolution, respectivement sur une période courte et une période longue ;
• recevoir des données physiologiques relatives au patient ;
• déterminer, en fonction des indices MC et ML, de leurs pentes PC et PL, et des données physiologiques relatives au patient, le besoin en composé médicamenteux devant être administré audit patient, cette étape comprenant une sous-étape de détermination d'une quantité dudit composé à administrer ponctuellement au patient, dit bolus, comprenant :
∘ la détermination d'une première variable de logique floue *C_{MC}*, comprise entre 0 et 1, à partir d'une fonction d'appartenance *F_{MC}* de l'indice MC,
∘ la détermination d'une deuxième variable de logique floue *C_{PC}*, comprise entre 0 et 1, à partir d'une fonction d'appartenance *F_{PC}* de l'indice PC,
∘ la détermination que le bolus est égal à une première quantité prédéterminée lorsque la somme de la première variable de logique floue *C_{MC}* et de la deuxième variable de logique floue *C_{PC}* est strictement supérieure à 1, et que le bolus est égal à zéro sinon
• déterminer une deuxième quantité dudit composé à administrer, inférieure à ladite première quantité prédéterminée, en fonction des indices MC et ML, de leurs pentes PC et PL, et des données physiologiques relatives au patient, à la suite de la détermination d'un bolus, lorsque le bolus est égal à zéro.

10. Dispositif de délivrance (300) d'un composé médicamenteux, comprenant un dispositif d'évaluation (200) selon la revendication 9, associé à des moyens de commande (240) et à un système de perfusion (250), de préférence électrique.

## Patentansprüche

1. Verfahren (100) zu der Bewertung des Bedarfs eines Patienten an einer Arzneimittelverbindung, das durch eine Bewertungsvorrichtung (200) implementiert werden soll, die Mittel (210) zu der Messung der Herzvariabilität des Patienten zwischen 0,15 Hz und 4 Hz, eine Datenverarbeitungs- und - aufzeichnungseinheit (220) und Mittel (230) zu der Aufzeichnung physiologischer Daten in Bezug auf den Patienten umfasst, wobei das Verfahren die folgenden Schritte umfasst:
• einen Schritt (110) des Messens der Herzvariabilität des Patienten zwischen 0,15 Hz und 4 Hz;
• einen Schritt des ersten Bestimmens (120) eines ANI-Index in Bezug auf den Schmerzgrad des Patienten durch die Datenverarbeitungs- und - aufzeichnungseinheit (220) anhand der gemessenen Herzvariabilitätsdaten;
• einen Schritt des zweiten Bestimmens (130), durch die Datenverarbeitungs- und -aufzeichnungseinheit (220), anhand des ANI-Index, eines MC-Index, der den Mittelwert des ANI-Index über einen kurzen Zeitraum darstellt, und eines ML-Index, der den Mittelwert des ANI-Index über einen langen Zeitraum darstellt, sowie der PC- und PL-Steigungen der MC- und ML-Indizes, wobei die MC- und ML-Indizes dem Schmerzgrad eines Patienten entsprechen, und die PC- und PL-Indizes bei dessen Entwicklung einen kurzen bzw. langen Zeitraum darstellen;
• einen Schritt des Aufzeichnens (140) physiologischer Daten in Bezug auf den Patienten durch die Mittel (230) zu der Aufzeichnung physiologischer Daten in Bezug auf den Patienten;
• einen Schritt des dritten Bestimmens (150) des Bedarfs des Patienten an einer Arzneimittelverbindung durch die Datenverarbeitungs- und - aufzeichnungseinheit (220), in Abhängigkeit von den MC- und ML-Indizes, deren PC- und PL-Steigungen und den physiologischen Daten in Bezug auf den Patienten, wobei der dritte Bestimmungsschritt einen Unterschritt (151) des Bestimmens einer ersten Menge der Verbindung umfasst, die dem Patienten pünktlich verabreicht werden soll, der sogenannte Bolus, umfassend:
∘ Bestimmen einer ersten Fuzzy-Logik-Variablen *C_{MC}* zwischen 0 und 1 anhand einer Zugehörigkeitsfunktion *F_{MC}* des MC-Index,
∘ Bestimmen einer zweiten Fuzzy-Logik-Variablen *C_{pc},* die zwischen 0 und 1 liegt, anhand einer Zugehörigkeitsfunktion *F_{PC}* des PC-Index,
∘ Bestimmen, dass der Bolus einer ersten vorbestimmten Menge der Verbindung gleich ist, wenn die Summe aus der ersten Fuzzy-Logik-Variablen *C_{MC}* und der zweiten Fuzzy-Logik-Variablen *C_{pc}* strikt größer als 1 ist, und dass der Bolus ansonsten gleich Null ist;
wobei der dritte Bestimmungsschritt (150) ferner einen Unterschritt (152) des Bestimmens einer zweiten Menge der zu verabreichenden Verbindung umfasst, die kleiner ist als die erste vorbestimmte Menge, die in Abhängigkeit von den MC- und ML-Indizes, deren PC- und PL-Steigungen und den physiologischen Daten in Bezug auf den Patienten bestimmt wird, wobei dieser Unterschritt (152) durch das Verfahren nach dem Unterschritt (151) des Bestimmens eines Bolus ausgeführt wird, wenn der Bolus gleich Null ist.

2. Verfahren (100) nach Anspruch 1, wobei die Zugehörigkeitsfunktion *F_{mc}* des MC-Index:
• gleich 1 ist, wenn der MC-Index kleiner oder gleich einem unteren Schwellenwert *S_{MCunten}* ist,
• gleich 0 ist, wenn der MC-Index größer oder gleich einem oberen Schwellenwert *S_{MCoben}* ist,
und einen monotonen abnehmenden Übergang zwischen 1 und 0 aufweist, wenn der MC-Index zwischen dem unteren Schwellenwert *S_{MCunten}* und dem oberen Schwellenwert *S_{MCoben}* liegt, und wobei die Zugehörigkeitsfunktion *F_{PC}* des PC-Index:
• gleich 1 ist, wenn der PC-Index kleiner oder gleich einem unteren Schwellenwert *S_{PCunten}* ist,
• gleich 0 ist, wenn der PC-Index größer oder gleich einem oberen Schwellenwert *S_{PCoben}* ist,
und einen monotonen abnehmenden Übergang zwischen 1 und 0 aufweist, wenn der PC-Index zwischen dem unteren Schwellenwert *S_{PCunten}* und dem oberen Schwellenwert *S_{PCoben}* liegt.

3. Verfahren (100) nach Anspruch 2, wobei die Zugehörigkeitsfunktion *F_{mc}* des MC-Index gemäß dem Ausdruck definiert ist: ${F}_{MC} = \left\{\begin{matrix}1 , & wenn MC \leq {S}_{MCunten} \\ \frac{MC - {S}_{MCoben}}{{S}_{MCunten} - {S}_{MCoben}} , & wenn {S}_{MCunten} < MC < {S}_{MCoben} \\ 0 , & wenn MC \geq {S}_{MCoben}\end{matrix}\right.$ und die Zugehörigkeitsfunktion *F_{PC}* des PC-Index gemäß dem Ausdruck definiert ist: ${F}_{PC} = \left\{\begin{matrix}1 , & wenn PC \leq {S}_{PCunten} \\ \frac{PC - {S}_{PCoben}}{{S}_{PCunten} - {S}_{PCoben}} , & wenn {S}_{PCunten} < PC < {S}_{PCoben} \\ 0 , & wenn PC \geq {S}_{PCoben}\end{matrix}\right.$

4. Verfahren (100) nach einem der Ansprüche 2 oder 3, wobei:
• der Schwellenwert *S_{MCunten}* zwischen 45 und 55 liegt und vorzugsweise gleich 50 ist,
• der Schwellenwert *S_{MCoben}* zwischen 58 und 68 liegt und vorzugsweise gleich 63 ist,
• der Schwellenwert *S_{PCunten}* zwischen -40 und -30 liegt und vorzugsweise gleich -35 ist,
• der Schwellenwert *S_{PCoben}* zwischen -30 und -20 liegt und vorzugsweise gleich -25 ist.

5. Verfahren (100) nach einem der Ansprüche 1 bis 4, wobei der Schritt (140) des Aufzeichnens physiologischer Daten in Bezug auf den Patienten ein Schritt des Aufzeichnens mindestens eines systolischen Blutdrucks (PAS) des Patienten durch das Mittel (230) zu der Aufzeichnung physiologischer Daten ist, das so konfiguriert ist, dass es mindestens einen systolischen Blutdruck (PAS) misst und aufzeichnet, wobei der Unterschritt (151) des Bestimmens eines Bolus durch das Verfahren durchgeführt wird, wenn der systolische Blutdruck des Patienten (PAS) größer oder gleich mindestens einem von einem ersten Schwellenwert *S*_{*PAS*1} für den systolischen Blutdruck und einem zweiten Schwellenwert *S*_{*PAS*2} für den systolischen Blutdruck ist.

6. Verfahren (100) nach Anspruch 5, wobei der erste Schwellenwert *S_{PAS1}* für den systolischen Blutdruck einem vorbestimmten Mittelwert für den systolischen Blutdruck entspricht, und der zweite Schwellenwert *S*_{*PAS*2} für den systolischen Blutdruck einem vor der Ausführung des Verfahrens gemessenen und aufgezeichneten systolischen Blutdruck des Patienten entspricht.

7. Verfahren (100) nach Anspruch 6, wobei der zuvor gemessene und aufgezeichnete systolische Blutdruck des Patienten einem während einer vorherigen Ausführung des Verfahrens gemessenen und aufgezeichneten systolischen Blutdruck des Patienten entspricht.

8. Verfahren (100) nach einem der Ansprüche 5 bis 7, ferner umfassend einen Schritt der proportionalen Regelung(145) einer zu verabreichenden Menge an Arzneimittelverbindung, der vor dem dritten Bestimmungsschritt (150) durch das Verfahren durchgeführt wird, wenn der systolische Blutdruck des Patienten (PAS) sowohl unter dem ersten Schwellenwert *S*_{*PAS*1} als auch unter dem zweiten Schwellenwert *S*_{*PAS*2} für den systolischen Blutdruck liegt, wobei der Regelungsschritt (145) das Bestimmen einer zu verabreichenden Menge an Arzneimittelverbindung, die einer zuvor verabreichten Menge an Arzneimittelverbindung entspricht, von der eine Menge an Arzneimittelverbindung subtrahiert wird, die direkt proportional zu einer Änderung des gemessenen und aufgezeichneten Werts des systolischen Blutdrucks (PAS) des Patienten ist, in Bezug auf einen vor der Ausführung des Verfahrens gemessenen und aufgezeichneten Wert des systolischen Blutdrucks des Patienten umfasst.

9. Vorrichtung zu der Bewertung (200) des Bedarfs eines Patienten an einer Arzneimittelverbindung, umfassend Mittel (210) zu der Messung der Herzvariabilität des Patienten zwischen 0,15 Hz und 4 Hz, eine Datenverarbeitungs- und -aufzeichnungseinheit (220) und Mittel zu der Aufzeichnung (230) physiologischer Daten in Bezug auf den Patienten, wobei die Verarbeitungs- und - aufzeichnungseinheit einen Prozessor (221) und einen Computerspeicher (222) umfasst, auf dem Anweisungen gespeichert sind, die den Prozessor für Folgendes konfigurieren:
• Empfangen von Daten zu der Herzvariabilität des Patienten zwischen 0,15 Hz und 4 Hz;
• Bestimmen eines ANI-Index in Bezug auf den Schmerzgrad des Patienten anhand der empfangenen Herzvariabilitätsdaten;
• Bestimmen, anhand des ANI-Index, eines MC-Index, der den Mittelwert des ANI-Index über einen kurzen Zeitraum darstellt, und eines ML-Index, der den Mittelwert des ANI-Index über einen langen Zeitraum darstellt, sowie der PC- und PL-Steigungen der MC- und ML-Indizes, wobei die MC- und ML-Indizes dem Schmerzgrad eines Patienten entsprechen, und die PC- und PL-Indizes bei dessen Entwicklung einen kurzen bzw. langen Zeitraum darstellen;
• Empfangen physiologischer Daten in Bezug auf den Patienten;
• anhand der MC- und ML-Indizes, deren PC- und PL-Steigungen sowie den physiologischen Daten in Bezug auf den Patienten, Bestimmen des Bedarfs an einer Arzneimittelverbindung, die dem Patienten verabreicht werden soll, wobei dieser Schritt einen Unterschritt des Bestimmens einer Menge der Verbindung umfasst, die dem Patienten pünktlich verabreicht werden soll, der sogenannte Bolus, umfassend:
∘ Bestimmen einer ersten Fuzzy-Logik-Variablen *C_{MC}*, die zwischen 0 und 1 liegt, anhand einer Zugehörigkeitsfunktion *F_{MC}* des MC-Index,
∘ Bestimmen einer zweiten Fuzzy-Logik-Variablen *C_{pc},* die zwischen 0 und 1 liegt, anhand einer Zugehörigkeitsfunktion *F_{PC}* des PC-Index,
∘ Bestimmen, dass der Bolus gleich einer ersten vorbestimmten Menge ist, wenn die Summe aus der ersten Fuzzy-Logik-Variablen *C_{MC}* und der zweiten Fuzzy-Logik-Variablen *C_{pc}* strikt größer als 1 ist, und dass der Bolus ansonsten gleich Null ist,
• Bestimmen einer zweiten Menge der zu verabreichenden Verbindung, die kleiner ist als die vorbestimmte erste Menge, in Abhängigkeit von den MC- und ML-Indizes, deren PC- und PL-Steigungen und den physiologischen Daten in Bezug auf den Patienten nach der Bestimmung eines Bolus, wenn der Bolus gleich Null ist.

10. Vorrichtung (300) zu der Abgabe einer Arzneimittelverbindung, umfassend eine Bewertungsvorrichtung (200) nach Anspruch 9, in Verbindung mit Steuermitteln (240) und einem Infusionssystem (250), das vorzugsweise elektrisch ist.

## Claims

1. Method (100) for assessing the needs of a patient for a drug compound, intended to be implemented by an assessment device (200) comprising means (210) for measuring the patient's cardiac variability between 0.15 Hz and 4 Hz, a data processing and recording unit (220), and means (230) for recording physiological data relating to the patient, the method comprising the following steps:
• a step (110) of measuring the cardiac variability of the patient between 0.15 Hz and 4 Hz;
• a step of first determining (120) by the data processing and recording unit (220), from the measured cardiac variability data, an ANI index relating to the pain level of the patient;
• a step of secondly determining (130) by the data processing and recording unit (220), from the ANI index, an MC index, representative of the mean of the ANI index over a so-called short period, and an ML index, representative of the average of the ANI index over a so-called long period, as well as the PC and PL slopes respectively of said MC and ML indices, the MC and ML indices corresponding to the pain level of a patient and the PC and PL indices to its change, over a short period and a long period respectively;
• a step of recording (140) physiological data relating to the patient, by means (230) for recording physiological data relating to the patient;
• a step of third determination (150) by the data processing and recording unit (220), according to the MC and ML indices, their slopes PC and PL, and physiological data relating to the patient, the need for a medicinal compound required by the patient, said third determination step comprising a sub-step (151) of determining a first amount of said compound to be administered to the patient in a single dose, referred to as a bolus, comprising:
∘ determining a first fuzzy logic variable *C_{MC}*, between 0 and 1, from a membership function *F_{MC}* of the MC index,
∘ determining a second fuzzy logic variable *C_{PC}*, between 0 and 1, from a membership function *F_{PC}* of the index PC,
∘ determining that the bolus is equal to a first predetermined amount of said compound when the sum of the first fuzzy logic variable *C_{MC}* and the second fuzzy logic variable *C_{PC}* is strictly greater than 1, and that the bolus is equal to zero otherwise;
said third determination step (150) further comprising a sub-step (152) of determining a second amount of said compound to be administered, less than said first predetermined amount, determined according to the indices MC and ML, their slopes PC and PL, and physiological data relating to the patient, this sub-step (152) being executed by the method following the sub-step (151) of determining a bolus when the bolus is zero.

2. Method (100) according to claim 1, wherein the membership function *F_{MC}* of the MC index is:
• equal to 1 when the MC index is less than or equal to a low threshold value *S_{MCLow}*,
• equal to 0 when the MC index is greater than or equal to a high threshold value *S_{MCHigh}*,
and has a monotonic decreasing transition between 1 and 0, when the MC index is between the low *S_{MCLow}* and high *S_{MCHigh}* threshold values, and wherein the PC index membership function *F_{PC}*is:
• equal to 1 when the PC index is less than or equal to a low threshold value *S_{PCLow}*,
• *S_{PCHigh}* equal to 0 when the PC index is greater than or equal to a high threshold value,
and has a monotonic decreasing transition between 1 and 0, when the PC index is between the low *S_{PCLow}* and high *S_{PCHigh}* threshold values.

3. Method (100) according to claim 2, wherein the membership function *F_{MC}* of the MC index is defined according to the expression: ${F}_{MC} = \left\{\begin{matrix}1 , & if MC \leq {S}_{MCLow} \\ \frac{MC - {S}_{MCHigh}}{{S}_{MCLow} - {S}_{MCHigh}} , & if {S}_{MCLow} < MC < {S}_{MCHigh} \\ 0 , & if MC \geq {S}_{MCHigh}\end{matrix}\right.$ and the PC index membership function *F_{PC}* is defined according to the expression: ${F}_{PC} = \left\{\begin{matrix}1 , & if PC \leq {S}_{PCLow} \\ \frac{PC - {S}_{PCHigh}}{{S}_{PCLow} - {S}_{PCHigh}} , & if {S}_{PCLow} < PC < {S}_{PCHigh .} \\ 0 , & if PC \geq {S}_{PCHigh}\end{matrix}\right.$

4. Method (100) according to any one of claims 2 or 3, wherein
• the threshold value *S_{MCLow}* is between 45 and 55, and preferably equal to 50,
• the threshold value *S_{MCHigh}* is between 58 and 68, and preferably equal to 63,
• the threshold value *S_{PCLow}* is between -40 and -30 and preferably equal to -35,
• the threshold value *S_{PCHigh}* is between -30 and -20, and preferably equal to - 25.

5. Method (100) according to any one of claims 1 to 4, wherein the step of recording (140) physiological data relating to the patient is a step of recording at least one systolic blood pressure (SBP) of the patient, by the means of recording (230) physiological data that are configured to measure and record at least one systolic blood pressure (SBP), the sub-step (151) of determining a bolus being performed by the method when the patient's systolic blood pressure (SBP) is greater than or equal to at least one of a first systolic blood pressure threshold value *S*_{*SBP*1} and a second systolic blood pressure threshold value *S*_{*SBP*2}.

6. Method (100) according to claim 5, wherein the first systolic blood pressure threshold value *S*_{*SBP*1} corresponds to a predetermined mean value of systolic blood pressure, and the second systolic blood pressure threshold value *S*_{*SBP*2} corresponds to a systolic blood pressure of the patient measured and recorded prior to performing said method.

7. The method (100) of claim 6, wherein the previously measured and recorded systolic blood pressure of the patient corresponds to a previously measured and recorded systolic blood pressure of the patient during a previous execution of said method.

8. Method (100) according to any one of claims 5 to 7, further comprising a step of proportional regulation (145) of an amount of drug compound to be administered, performed by the method upstream of the third determination step (150), when the patient's systolic blood pressure (SBP) is strictly lower than both said first threshold value *S*_{*SBP*1} and said second systolic blood pressure threshold value *S*_{*SBP*2},
said regulation step (145) comprising determining an amount of drug compound to be administered corresponding to an amount of previously administered drug compound, from which an amount of drug compound directly proportional to a change in the measured and recorded value of the patient's systolic blood pressure (SBP) is subtracted, with respect to a value of the patient's systolic blood pressure measured and recorded prior to the execution of said method.

9. Device (200) for assessing the needs of a patient for a drug compound, comprising means (210) for measuring the patient's cardiac variability between 0.15 Hz and 4 Hz, a data processing and recording unit (220), and means (230) for recording physiological data relating to the patient, the processing and recording unit comprising a processor (221) and a computer memory (222) storing instructions configuring the processor to:
• receive data on the patient's cardiac variability between 0.15 Hz and 4 Hz;
• determine, from the received cardiac variability data, an ANI index relative to the level of pain of the patient;
• determine, from the ANI index, a MC index representative of the average of the ANI index over a so-called short period, and an ML index, representative of the average of the ANI index over a so-called long period, as well as the PC and PL slopes respectively of said MC and ML indices, the MC and ML indices corresponding to the pain level of a patient and the PC and PL indices as it evolves, over a short period and a long period respectively;
• receive physiological data relating to the patient;
• determine, based on the MC and ML indices, their PC and PL slopes, and physiological data relating to the patient, the need for a drug compound to be administered to said patient, this step comprising a sub-step of determining an amount of said compound to be administered punctually to the patient, called a bolus, comprising:
∘ determine a first fuzzy logic variable *C_{MC}*, between 0 and 1, from a membership function *F_{MC}* of the MC index,
∘ determine a second fuzzy logic variable *C_{PC}*, between 0 and 1, from a membership function *F_{PC}* of the index PC,
∘ determine that the bolus is equal to a first predetermined amount when the sum of the first fuzzy logic variable *C_{MC}* and the second fuzzy logic variable *C_{PC}* is strictly greater than 1, and that the bolus is equal to zero otherwise
• determine a second amount of said compound to be administered, less than said first predetermined amount, according to the MC and ML indices, their PC and PL slopes, and physiological data relating to the patient, following the determination of a bolus, when the bolus is equal to zero.

10. Device (300) for delivering a drug compound, comprising an assessment device (200) according to claim 9, associated with control means (240) and an infusion system (250), preferably electrical.
